Europäisches Patentamt

European Patent Office

Office européen des brevets

(19)

(11) Publication number: **0 280 004**
**B1**

## EUROPEAN PATENT SPECIFICATION

(12)

(45) Date of publication of the patent specification:
**16.08.89**

(51) Int. Cl.⁴: **A61F 2/60**

(21) Application number: **87850062.8**

(22) Date of filing: **25.02.87**

(54) Foot prosthesis.

(43) Date of publication of application:
**31.08.88 Bulletin 88/35**

(45) Publication of the grant of the patent:
**16.08.89 Bulletin 89/33**

(84) Designated Contracting States:
**AT BE CH DE FR GB IT LI LU NL SE**

(56) References cited:
**FR-E- 25 322**
**GB-A- 534 057**

(73) Proprietor: **Ljungblad, Yngve, Ringvägen 84,
S-902 54 Umea(SE)**

(72) Inventor: **Ljungblad, Yngve, Ringvägen 84,
S-902 54 Umea(SE)**

(74) Representative: **Onn, Thorsten et al, AB STOCKHOLMS
PATENTBYRA Box 3129, S-103 62 Stockholm(SE)**

ACTORUM AG

## Description

This invention relates to a foot prosthesis as set out in the prior art portion of claim 1.

Foot prostheses of these types have been known for a long time and are usually so made that their carriers can use usual shoes having a definite heel height. These known prostheses are felt pleasant to the carrier only with this definite heel height and at any other heel height of the shoe or when walking without shoe they are felt to be unpleasant and almost unusable. This means that carriers of known prostheses are compelled to use shoes with only one definite heel height which is a great disadvantage i.a. for the possibilities of a prosthesis carrier to choose freely among the different types of shoes available in the market for persons not carrying a prosthesis.

It is therefore the object of this invention to provide a foot prosthesis not having the above-mentioned shortcomings but being so constituted that it is adaptable to varying heel heights and consequently always will be felt to be pleasant for the carrier independently of heel height and if the carrier walks with or without shoes. This is realized according to the invention by the features of the characterizing portion of claim. Adaptation of the invention prosthesis is always made possible to the type of shoes carried by the prosthesis carrier on a certain occasion and to walks without shoes.

It is advantageous to form the foot sole and the heel in one piece of the same elastic material enabling turning of the heel relative to the foot sole and to have only one part of the foot sole fixedly connected to the central portion so that the heel can move relative to the part of the foot sole fixedly connected to the central portion of the foot prosthesis.

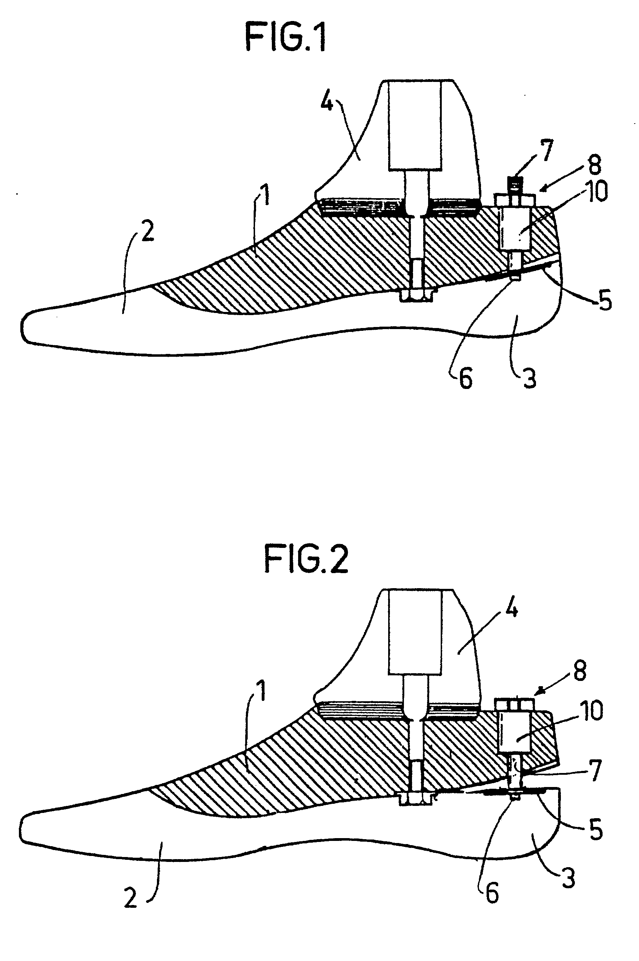

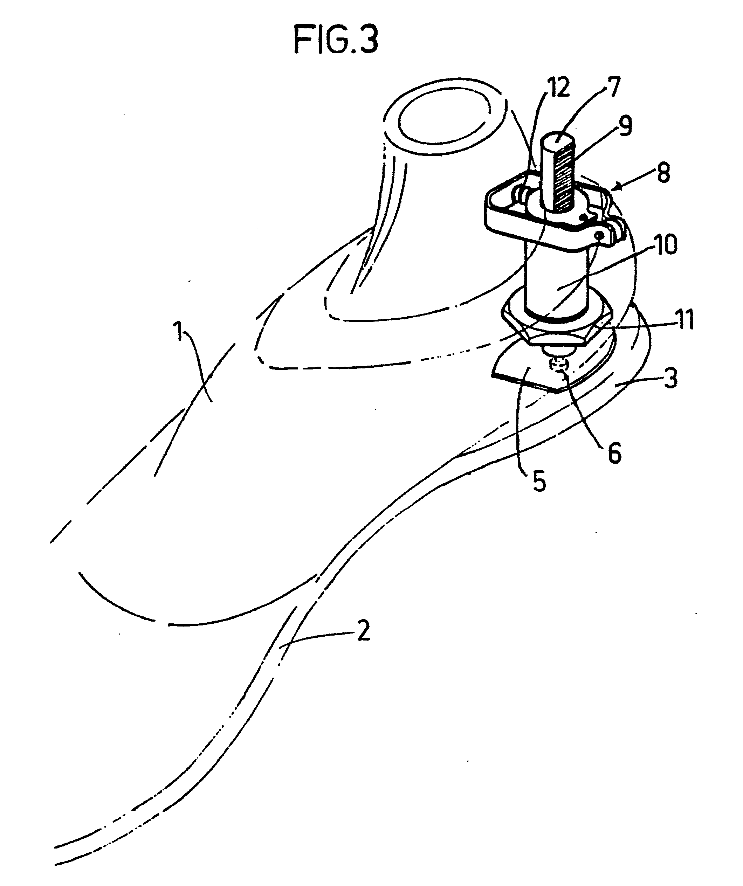

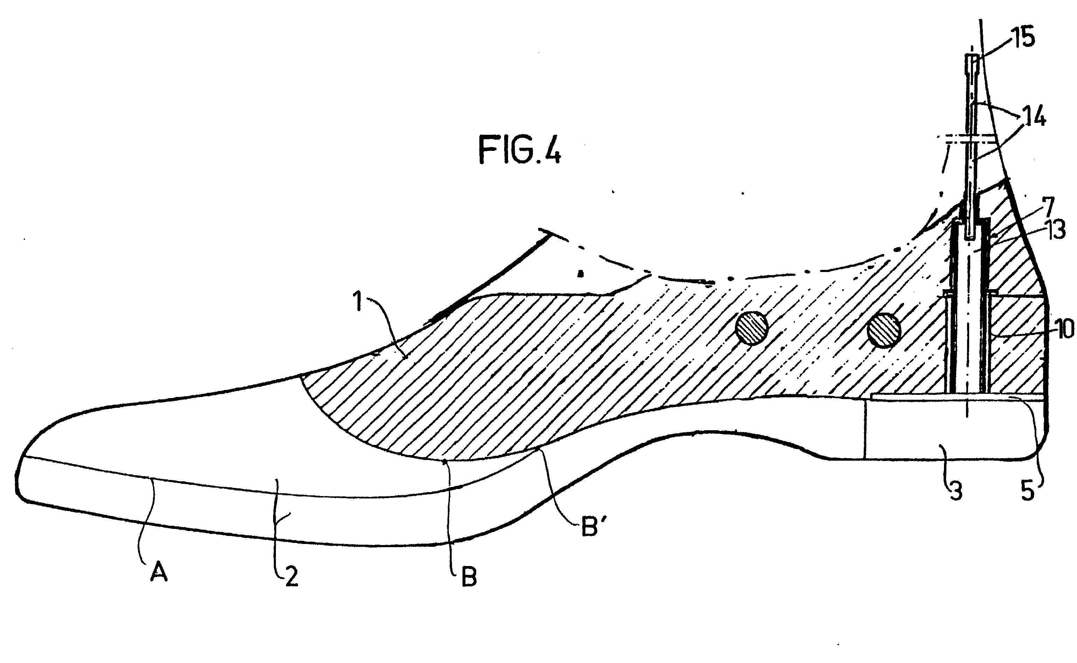

The invention is described below in greater detail with reference to the enclosed drawings in which Fig. 1 shows a section of an embodiment of the present foot prosthesis set for use together with a shoe of a certain heel height, Fig. 2 shows a section of the same prosthesis design as Fig. 1 but adjusted to be used together with a shoe of another heel height than in Fig. 1, Fig. 3 shows a device for adjusting the position of the heel relative to the foot sole and for locking the heel in the set position and Fig. 4, finally, shows an alternative design of the adjusting mechanism of the heel.

The present foot prosthesis comprises a central portion 1 of a stiff material arranged as an ankle, e.g. wood with glued wooden plugs to prevent cracking, a foot sole 2 with heel 3 and an adapter 4 for connecting the foot prosthesis to the lower leg portion or a leg prosthesis. In the embodiment shown the foot sole 2 and the heel 3 are made in one piece of an elastic material, e.g. plastic or rubber having a hardness of about 40-60 shore, the part lying above the line A in Fig. 4 preferably being harder than the lower part being the very foot sole. At its front part the foot sole is fixedly connected to the central portion up to the point B or B'. Otherwise the foot sole 2 together with its heel 3 is loosely arranged relative to the central portion 1.

A pressure absorbing disc 5 or the like is fixedly arranged in the heel 3 and the lower part of an adjusting means 7 in the form of a rod is attached to the disc 5 and consequently the heel 3 by means of a fastening means, e.g. a screw 6 passing the disc. In its longitudinal direction the adjusting means 7 is movably arranged in a sleeve 10 connected to a locking means 8, said sleeve being fixedly mounted in a hole in the rear part of the central portion 1. The adjusting means 7 can be immovably locked by the locking means 8 relative to the central portion 1 in different adjusting positions and, thus, the adjusting means 7 can be displaced in unlocked position in its longitudinal direction for adjusting the heel 3 of the prosthesis in the intended position relative to the front part of the foot sole fixedly connected to the central portion 1. In the example shown this adjustment is made possible thanks to the fact that the foot sole 2 and the heel 3 are formed in one piece of an elastic material but in other designs not shown in detail the part of the foot sole which together with the heel 3 is not connected to the central portion 1 can be articulatedly connected to the part of the foot sole fixedly connected to the central portion through a link connection of e.g. hinge type.

The adjustment of the heel relative to the central portion 1 can be carried out in many different ways within the scope of the present invention. A spring not shown on the drawings can for instance be adapted between the heel 3 and the sleeve 10 which is preferably provided at its lower portion with an adjusting ring 11 preferably threaded on the sleeve 10 and lying free of the central portion 1, as shown in Fig. 3. Thus, said spring between the adjusting ring and the disc 5 in the heel 3 can be adjustably clamped to press the heel at unlocked adjusting means 7 in a direction away from the central portion 1 to an outer end position in which the heel 3 is maximally displaced from the central portion 1. By moving the latter, for instance manually or by means of a pressure on the heel 3, towards the central portion 1 it can be simply adjusted to the desired position relative to the part of the foot sole fixedly connected to the central portion 1, after which the locking means 8 is locked to maintain the heel in the adjusted position. Thus, in this way an exact adaptation to the heel height of the shoe which is intended to be carried can be easily made.

In order to lock the adjusting means 7 in an adjusted position the rod-shaped adjusting means 7 in the embodiment shown in Figs. 1-3 is formed with a plane side which can extend from the upper portion of the adjusting means along the whole adjusting means or only along part of its length and which side is provided with a toothing 9 while the very locking means is arranged to engage this toothing 9 of the adjusting means 7 in locking position and to provide in this way an immovable connection between the central portion 1 and the heel 3 of the prosthesis. In order to secure the engagement between the locking means 8 and the toothing 9 a spring 12 is arranged pressing the locking means 8 against the toothing 9 at locking. By moving the toothing and the locking means from each other against the action of the spring 12 the adjusting means 7 is thus released so that it can be moved in its longitudinal direction for adjusting the heel in the intended position.

In order to achieve displacement of the adjusting means 7 and consequently adjustment of the heel 3 relative to the foot sole with the part fixedly connected to the central portion the adjusting means 7 is formed in an advantageous embodiment as a stud 13 (see Fig. 4) with threads for coaction with a sleeve 10 provided with corresponding threads and immovably anchored in a hole in the rear part of the central portion. The adjusting means 7 is turnable but not axially movably connected with the disc 5 fixedly arranged in the heel 3. The heel 3 is adjusted relative to the foot sole 2 by turning the adjusting means 7 in its sleeve 10 provided with threads, and to reduce the force required for turning the adjusting means 7 its threads and the threads of the sleeve should have a relatively small pitch. These threads should also be self-restrictive, locking of the adjusting means 7 in the adjusting position automatically being obtained. Thus, when turning the adjusting means 7 this is moved in longitudinal direction and the position of the heel relative to the part of the foot sole fixedly connected to the central portion 1 can be adjusted in the required degree for adaptation of the foot prosthesis to the heel height of the shoe to be carried.

In order to make it easier for the carrier of the prosthesis to adjust the foot prosthesis a flexible wire 14 or the like, which is resistant to turning, is fixedly connected to the adjusting means 7, see Fig. 4, and is provided with a turning knob 15 at its upper end. The wire 14 should preferably have such a length that the turning knob 15 can be placed in such a position, for instance at beneath or above the knee joint of the leg prosthesis, that the carrier of the prosthesis can reach it to adjust the foot prosthesis in the desired position without having to bend.

Of course it is also possible within the scope of the invention to achieve the displacement of the adjusting means formed as a stud 13 relative to the central portion 1 of the foot prosthesis by arranging a nut or the like coacting with the threads of the adjusting means and turning this instead of the screw 13.

The heel 3 of the foot prosthesis can also be adjusted by an adjusting means arranged between the central portion 1 and the heel 3 of the foot prosthesis, said adjusting means having the form of a pair of bellows or being embodied in another way so that its thickness can be changed, for instance hydraulically or pneumatically or by means of a simple mechanism.

In the illustrative example shown and described the heel 3 is shown to be pivotally arranged relative to the part of the foot sole 2 fixedly connected to central portion 1, but it is also possible within the scope of the invention to arrange the heel so that it can be moved vertically relative to the central portion 1, the whole foot sole 2 except the heel being fixedly connected to the central portion 1, and suitable guides being arranged to prevent the heel from turning.

The invention is not restricted to what has been described above and shown on the drawings but it can be changed supplemented and modified in several different ways within the scope of the inventive data defined in the claims.

## Claims

1. Foot prosthesis comprising a foot sole (2), a heel (3) and a central supporting portion (1) provided with an adapter (4) for connecting the foot prosthesis to a lower leg prosthesis and supporting the foot sole (2) and the heel (3), **characterized** in that part of the foot sole (2) is fixedly connected to the central portion (1) of the prosthesis and the heel (3) is movably supported relative to that part the heel being thereby connected to the central portion as well as the adapter (4), and in that the heel (3) is coupled to the rear part of the central portion through an adjusting means (7) associated with the central portion and the heel for moving the heel vertically towards or away from the central portion and consequently for adjusting the heel (3) to a desired vertical position relative to the part of the foot sole fixedly connected to the central portion (1), said adjusting means (7) being lockable in each adjusted position to retain the heel (3) in its position adjusted relative to said portion of the foot sole.

2. Foot prosthesis according to claim 1, **characterized** in that the foot sole (2) and the heel (3) are made of an elastically flexible material and that at least a front portion of the foot sole (2) is fixedly connected with the central portion (1) of the prosthesis.

3. Foot prosthesis according to claim 2, **characterized** in that the heel (3) and the foot sole (2) are made in one piece of the same elastic material.

4. Foot prosthesis according to claim 2, **characterized** in that the heel (3) and the part of the foot sole (2) integral with the heel and loosely supported relative to the central portion is pivotally connected in a joint with the portion of the foot sole fixedly connected to the central portion (1).

5. Foot prosthesis according to claim 1 or 2, **characterized** in that the heel (3) is formed separately from the foot sole (2) fixedly connected to the central portion (1) and is linearly movably supported by the adjusting means (7), guides being arranged to prevent the heel (3) from turning relative to the central portion (1).

6. Foot prosthesis according to any one of the preceding claims, **characterized** in that one end of the adjusting means (7) is connected to a pressure absorbing and distributing plate (5) fixedly arranged in the heel (3).

7. Foot prosthesis according to any one of the preceding claims, **characterized** in that the adjusting means comprises a rod movable in a sleeve (10) in the rear part of the central portion and a spring arranged between the central portion (1) and the heel and allowing a relative motion in the unlocked position of the rod between the central portion (1) and the heel of the prosthesis, a locking means (8) for locking the rod in the adjusted position being arranged in connection with the sleeve (10) surrounding the rod for an engaging coaction with a toothing

(9) on one side of the rod by the action of a spring (12).

8. Foot prosthesis according to any one of the preceding claims 1-6, **characterized** in that the adjusting means (7) comprises a rod (13) threaded along its whole length or parts of its length which is rotatably connected with the plate (5) arranged in the heel and extends through and cooperates with a nut means (10) provided with corresponding threads to effect the displacement of the rod by turning either the rod or the nut means.

9. Foot prosthesis according to claim 8, **characterized** in that the threads of the adjusting means have a small pitch and are self-restrictive for locking the adjusting means (7) in the set position.

10. Foot prosthesis according to claim 8 or 9, **characterized** in that a wire (14) or the like, which is resistant to twisting, connects the prosthesis carrier to that part of the parts of the adjusting means which is arranged to be turned for providing the adjustment of the heel.

## Patentansprüche

1. Fussprothese, die eine Fussohle (2), einen Fersen (3) und eine zentrale Stützpartie (1) mit einem Anschlussteil (4) zur Verbindung der Fussprothese mit einer Unterbeinprothese enthält, und die Fussohle (2) und die Ferse (3) stützt, dadurch gekennzeichnet, dass ein Teil der Sohle (2) fest mit der Mittelpartie (1) der Prothese verbunden ist und dass die Ferse (3) gegenüber diesem Teil beweglich gestützt wird und dass die Ferse dadurch mit der Mittelpartie sowie dem Anschlussteil (4) verbunden ist, und dass die Ferse (3) an den Rückteil der Mittelpartie mittels eines, der Mittelpartie zugehörenden Stellorgans (7) angeschlossen ist um die Ferse lotrecht in Richtung zur oder von der Mittelpartie weg zu bewegen und um die Ferse (3) in einer gewünschten Vertikallage bezüglich der Partie der fest mit der Mittelpartie (1) verbundenen Fussohle einzustellen, wobei das Stellorgan (7) in jeglicher Stellage verschliessbar ist um die Ferse (3) in ihrer korrigierten Lage bezüglich der genannten Partie der Fussohle festzuhalten.

2. Fussprothese gemäss Patentanspruch 1, dadurch gekennzeichnet, dass die Fussohle (2) und die Ferse (3) aus einem elastisch biegsamen Werkstoff hergestellt sind, und dass zumindestens ein Vorderteil der Fussohle (2) fest mit der Mittelpartie der Prothese verbunden ist.

3. Fussprothese gemäss Patentanspruch 2, dadurch gekennzeichnet, dass die Ferse (3) und die Fussohle (2) aus einem Stück aus demselben elastischen Werkstoff bestehen.

4. Fussprothese gemäss Patentanspruch 2, dadurch gekennzeichnet, dass die Ferse (3) und der Teil der Fussohle (2), der aus einem Stück mit der Ferse besteht und lose in bezug auf die Mittelpartie gestützt wird, in einem Gelenk mit dem mit der Mittelpartie fest verbundenen Teil der Fussohle drehbar verbunden ist.

5. Fussprothese gemäss Patentanspruch 1 oder 2, dadurch gekennzeichnet, dass die Ferse (3) getrennt von der Fussohle (2) ausgeführt ist, die fest mit der Mittelpartie (1) verbunden ist, und linienförmig beweglich vom Stellorgan (7) gestützt wird, wobei Steuerungen angeordnet sind um ein Drehen der Ferse (3) in Bezug auf die Mittelpartie (1) zu verhindern.

6. Fussprothese gemäss einem der vorhergehenden Patentansprüche, dadurch gekennzeichnet, dass das eine Ende des Stellorganes (7) mit einer druckabsorbierenden und druckverteilenden, fest in der Ferse (3) angeordneten Platte (5) verbunden ist.

7. Fussprothese gemäss einem der vorhergehenden Patentansprüche, dadurch gekennzeichnet, dass das Stellorgan eine in einer Büchse (10) bewegliche Stange im Rückteil der Mittelpartie umfasst, und dass eine Feder zwischen der Mittelpartie (1) und der Ferse angeordnet ist und eine relative Bewegung in unverriegelter Lage der Stange zwischen der Mittelpartie (1) und der Ferse der Prothese zulässt, wobei ein Riegel (8) zur Verriegelung der Stange in der korrigierten Lage in Verbindung mit einer Verzahnung (9) auf einer Seite der Stange unter Einwirkung der Feder (12) steht.

8. Fussprothese gemäss einem der vorhergehenden Patentansprüche 1–6, dadurch gekennzeichnet, dass das Stellorgan (7) eine entlang ihrer gesamten Länge oder einem Teil derselben mit Gewinde versehene Stange (13) umfasst, die drehbar mit der in der Ferse angeordneten Platte (5) verbunden ist und sich durch ein mit entsprechendem Gewinde versehenes Mutterteil (10) erstreckt und mit diesem zusammenwirkt um die Verrückung der Stange durch Drehen entweder der Stange oder des Mutterteiles zu bewerkstelligen.

9. Fussprothese gemäss Patentanspruch 8, dadurch gekennzeichnet, dass das Gewinde des Stellorgans eine geringe Steigung aufweist und selbsthemmend wirkt um das Stellorgan (7) in der Endposition zu verrriegeln.

10. Fussprothese gemäss Patentanspruch 8 oder 9, dadurch gekennzeichnet, dass ein verdrehfester Draht oder ähnliches den Prothesenträger mit der Partie der Teile des Stellorgans verbindet, der vorgesehen ist für die Einstellung der Ferse gedreht zu werden.

## Revendications

1. Prothèse du pied comprenant une plante (2) du pied, un talon (3) et une partie centrale portante (1) munie d'un adaptateur (4) permettant de relier la prothèse du pied à une prothèse de la jambe et supportant la plante (2) et le talon (3), caractérisée en ce qu'une partie de la plante (2) est solidaire de la partie centrale (1) de la prothèse et que le talon (3) est supporté mobile par rapport à ladite partie, le talon étant ainsi relié à la partie centrale et, par là, à l'adaptateur (4), et en ce que le talon (3) est accouplé à la partie arrière de la partie centrale par l'intermédiaire d'un organe (7) de réglage relié à la partie centrale et au talon et permettant de déplacer le talon verticalement en direction de la partie centrale ou dans le sens opposé, assurant ainsi un ajustage du talon (3) en une position verticale voulue par rapport à la partie de la plante solidaire de la partie centrale (1), ledit organe (7) de réglage pouvant être bloqué dans chaque position choisie pour maintenir le talon (3) dans sa position réglée par rapport à ladite partie de la plante du pied.

2. Prothèse du pied selon la revendication 1, caractérisée en ce que la plante (2) du pied et le talon (3) sont réalisés en une matière élastiquement flexible et qu'une partie avant au moins de la plante (2) du pied est solidaire de la partie centrale (1) de la prothèse.

3. Prothèse du pied selon la revendication 2, caractérisée en ce que le talon (3) et la plante (2) du pied sont réalisés d'une pièce d'une même matière élastique.

4. Prothèse du pied selon la revendication 2, caractérisée en ce que le talon (3) et la partie de la plante (2) réalisée d'une pièce avec le talon et lâchement supportée par la partie centrale sont reliés basculants, au moyen d'une articulation, à la partie de la plante solidaire de la partie centrale (1).

5. Prothèse du pied selon l'une des revendications 1 et 2, caractérisée en ce que le talon (3) est séparé de la plante (2) solidaire de la partie centrale (1) et qu'il est supporté linéairement mobile par l'organe (7) de réglage, des guides étant disposés pour empêcher le talon (3) de tourner par rapport à la partie centrale (1).

6. Prothèse du pied selon une quelconque des revendications précédentes, caractérisée en ce que l'une des extrémités de l'organe (7) de réglage est reliée à une plaque (5) disposée fixe dans le talon (3) et servant à absorber et à répartir la pression.

7. Prothèse du pied selon une quelconque des revendications précédentes, caractérisée en ce que l'organe de réglage comprend une tige mobile dans une douille (10) disposée dans la partie arrière de la partie centrale et un ressort disposé entre la partie centrale (1) et le talon et permettant, à l'état non bloqué de la tige, un mouvement relatif du talon par rapport à la partie centrale (1) de la prothèse, le blocage de la tige dans la position choisie étant assuré par un organe (8) de blocage disposé contigu à la douille (10) entourant la tige et agencé pour venir en prise, grâce à l'action d'un ressort (12), avec une denture (9) réalisée d'un côté de la tige.

8. Prothèse du pied selon une quelconque des revendications 1 à 6, caractérisée en ce que l'organe (7) de réglage comporte une tige (13) filetée sur toute sa longueur ou sur certaines parties de celle-ci, reliée rotative à la plaque (5) disposée dans le talon et s'étendant par un organe (10) taraudé en conséquence pour faire fonction d'écrou coopérant avec la tige (13), de manière à permettre le déplacement de la tige par rotation de celle-ci ou de l'organe faisant fonction d'écrou.

9. Prothèse du pied selon la revendication 8, caractérisée en ce que le filet de l'organe de réglage présente un pas faible et qu'il est à retenue automatique pour assurer le blocage de l'organe (7) de réglage dans la position choisie.

10. Prothèse du pied selon l'une des revendications 8 et 9, caractérisée en ce qu'un câble flexible (14) ou équivalent, rigide en torsion, permet au porteur de la prothèse d'agir sur celle des parties de l'organe de réglage qu'il faut tourner pour ajuster la position du talon.

# FIG.1

# FIG.2

# FIG.3

FIG.4

EP 0 280 004 B1